(19) Europäisches Patentamt
European Patent Office
Office européen des brevets

(11) **EP 1 997 469 B1**

(12) **EUROPEAN PATENT SPECIFICATION**

(45) Date of publication and mention
of the grant of the patent:
**03.08.2011 Bulletin 2011/31**

(51) Int Cl.:
*A61H 31/00* *(2006.01)*

(21) Application number: **08157253.9**

(22) Date of filing: **30.05.2008**

(54) **Monitoring of chest compressions**

Überwachung von Thoraxkompressionen

Surveillance de compressions thoraciques

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **31.05.2007 GB 0710460**

(43) Date of publication of application:
**03.12.2008 Bulletin 2008/49**

(73) Proprietor: **Laerdal Medical AS
4002 Stavanger (NO)**

(72) Inventor: **Nysæther, Jon
N-4041, Hafrsfjord (NO)**

(74) Representative: **Onsagers AS
Universitetsgaten 7
P.O. Box 6963
St. Olavs plass
0130 Oslo (NO)**

(56) References cited:
**WO-A-00/27334          WO-A-02/15836
WO-A-2007/129908**

**Description**

The present invention relates to a device for monitoring chest compressions.

CPR feedback systems have recently gained attention as a method for improving the quality of CPR on a cardiac arrest patient. One typical feature of such systems is to measure the compression depth and rate during chest compressions, compare with accepted Guideline limits (American Heart Association 2005) and give verbal or visual feedback to the rescuer if, for instance, compression depth does not meet the accepted value of 38 mm - 50 mm (1.5 - 2 inches).

A system for giving feedback on compressions typically consists of a sensor pad to be placed on the patient's chest. The sensor pad may contain an accelerometer and optionally a force sensor. The compression depth measurement is usually based on double integration of acceleration. An additional force measurement can help in signal processing to remove accelerometer offset, and can also be used to give feedback on incomplete release, as disclosed in EP-1491176 A1.

WO-02/15836 describes a method for determining displacement of a region of a patients' chest to which force is applied so as to cyclically compress and decompress the chest and administer thereby CPR to the patient comprising determining displacement of the chest region by double integrating over time acceleration of the chest region and correcting integration constants using information independent of the integration. Visual means for displaying the performance of the CPR.

However, although current Guidelines specify a depth range for compressions, there is no direct relationship between compression depth and CPR efficacy that has been proven to be valid for all individuals.

Human bodies take various forms and sizes, and it is not logical that the same compression depth will be equally efficient in a large as in a small individual. This uncertainty is qualitatively accounted for in adult CPR Guidelines, where it is acknowledged that a larger individual may require more and a smaller individual may require less compression depth than the recommended value. For children, no definite depth target exist, and the rescuer is advised to compress a certain portion of the chest height of the cardiac arrest patient (American Heart Association 2005)

However, the acceleration based technology of most CPR feedback systems, including the cited documents above, only measures the stroke length of compressions. The technology is therefore not suitable to assess the size of the patient and compensate compression depth accordingly. In addition, if the patient is placed on a compliant mattress, current systems will detect the entire movement of the chest surface and not only the dimensional change of the chest which is relevant for CPR. Thus, the depth reported by the feedback system will be too large, and the rescuer may falsely believe that she is compressing deep enough.

Many feedback systems give a warning if the rescuer does not release all force from the chest during compressions. This feedback is often based on a measurement of the minimum force alone, or the force in combination with a crude estimation of "chest compliance" to estimate compression depth such as in patent application EP-1491176 A1. However, since the force/depth characteristics for small displacements of the chest vary significantly among individuals, and because the stiffness generally increases progressively with depth, the actual leaning depth associated with such measurements may be quite uncertain.

The invention has as an object to provide a device for giving individualized feedback on the magnitude of compressions delivered, so that the threshold for giving feedback is based on characteristic properties of the chest and also on the properties of the backing surface.

When performing chest compressions the chest must reach a predetermined level of compression before the compression force is transmitted to the blood vessels and blood circulation is achieved. In the context of the present application, a level of compression (expressed by a determined force exerted on the chest by and/or displacement of a compression member) which leads to adequate blood circulation will be referred to as "efficient compression".

Several models have been provided to simulate the chest behaviour under compression. Curves representing force vs. displacement show that this relation is linear for small deflections and non-linear for larger deflections. For low deflections, most of the force is used only to bend the rib cage, and this linear force is therefore not efficient in pressing on the heart and generating blood pressure. Only for higher compression depths, where the force/displacement relationship becomes non-linear, there is actually a generation of blood pressure and resulting flow. It can therefore be argued that it is only the non-linear part of the compression force that is efficient in generating adequate circulation.

Since the depth or force at which the force/depth relationship (i.e. stiffness) becomes non-linear varies from individual to individual, a measurement of chest compression based mainly on displacement of a compression member (stroke length) and/or compression force on the patient will not be able to appropriately determine whether a compression is "efficient" in generating circulation.

The invention takes this into consideration by providing a device for monitoring chest compressions performed by means of a compression member. The device comprises:

a first sensor arranged for measuring force exerted by the compression member,
a second sensor arranged for measuring displacement of the compression member, and a processor arranged for providing a chest stiffness function representing the relationship between force and displacement based on values derived from the measured force and the measured displacement, and for analyzing linearity of the chest stiffness function.

The device further comprises means for providing an indication of the chest compression efficacy based on the results of said linearity analysis, where the degree of non-linearity determines if a chest compression is indicated as efficient or non-efficient.

The invention is characterized by the features mentioned in the patent claims.

In an embodiment the invention comprises providing an indication of efficient chest compression based on the results of said linearity analysis, where the degree of non-linearity determines if a chest compression is indicated as efficient or non-efficient. In one embodiment, determination of non-linearity which exceeds a given threshold will lead to indication of efficient chest compression and determination of linearity or non-linearity which does not exceed said threshold will lead to indication of non-efficient chest compression.

According to the invention the non-linear stiffness of the chest under compression is determined based on force and depth measurements performed e.g. with a CPR sensor logging compression force and depth, and giving feedback according to a predetermined set of rules. The non-linear properties are assessed by measuring the compression depth and force during CPR.

The invention has as an object to provide an indication of efficient compressions based on displacement and force measurement.

The invention relies on the assumption that the non-linear part of the force vs. displacement curve is related to the efficacy of the chest compressions.

The invention will now be illustrated by means of examples shown in the figures, where:

figure 1 shows how a measured force/depth curve can be parameterized,
figure 2 illustrates how the non-linear parts of the force and depth are defined,
figure 3 shows use of leaning thresholds,
figure 4 illustrates curves for different patient types,
figure 5 shows curves for patients lying on different surfaces.

The elastic force-depth curve and damping can be calculated using e.g. the method described in Arbogast et al (2006). The method is briefly summarized as follows:

Force and depth are measured synchronously, and depth is differentiated to find velocity; for instance by subtracting two subsequent depth samples and dividing by the difference in sampling time. Due to the damping properties of the chest, the force at a given depth during compression (F1) will differ from the force at the same depth during decompression (F2). Assume that the difference (hysteresis) in force F1-F2 is caused by a damping force (Fd) being proportional to the speed of the chest. Based on the measured speed (v) and force difference (F1-F2), a damping constant can be calculated. The elasticity (stiffness) of the rib cage, assumed to being equal for compression and decompression, can now be found by subtracting the damping force from the measured force. Leaning depth is found by measuring the leaning force and the stiffness for low deflections, for instance for depths up to 10 mm.

For the following discussion, we will define a chest stiffness function F(x), a stiffness k(x) and an incremental chest stiffness g(x), where F(x) is the elastic part of the compression force and x is compression depth relative to the neutral position of the chest. The neutral position is the position to where the chest will recoil and come to rest if no force is applied:

$$\text{Stiffness: } k(x) = F(x)/x$$

$$\text{Incremental stiffness: } g(x) = dF(x)/dx$$

According to the invention linearity of the chest stiffness function F(x) is analyzed to monitor chest compressions.

The chest stiffness function can be determined in several ways, e.g.:

1) In one embodiment of the invention non-linearity is analyzed by calculating the progressivity of the chest, here defined as the ratio of stiffness or force measured at two different levels of compression. Tomlinson et al (2006) defined progressivity as $\Psi = F38/2F19$, where F38 and F19 are the forces required to reach 38 and 19 mm depth, respectively.

Since the force at zero compression depth by definition is 0, the ratio of the force measured at two different depths will in essence be related to the second derivative of the force with respect to depth or incremental stiffness. It is also possible to combine measurements of three or more force-depth points to estimate higher order derivatives of force or other, related measures.

The force at each level can for instance be calculated as the average of the corresponding measured forces from a predetermined number of previous compressions.

2) Another possibility of determining non-linearity of the chest stiffness function is to define it in terms of a depth at which the stiffness k(x) or the incremental stiffness g(x) have increased by a certain factor relative to the value at low deflections.

3) Yet another method of determining non-linearity of the chest stiffness function is to fit a parameterized function to the measured force-depth curve, for instance a polynomial, exponential or spline function. The parameters describing the function can then be calculated from previous compressions, temporarily stored by the system and used to calculate feedback relevant parameters.

The force-depth relationship can for instance be parameterized in the following ways:

By the function

$F(x) = k_1x + k_2/2x^2$ , which is characterized by the parameters $k_1$ and $k_2$.
Here, $k_2$ is an calculated second derivative of the chest stiffness function.

by the function

$F(x) = F0 (\exp(x/x0)-1)$, which is characterized by the parameters F0 and x0.

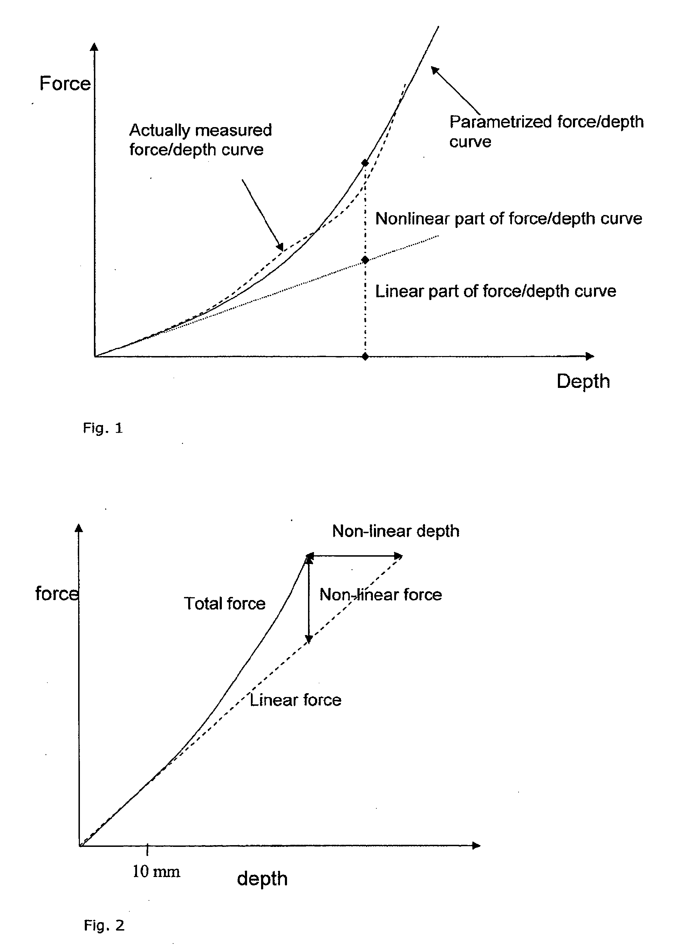

Figure 1 shows how a measured force/depth curve can be parameterized, for instance using a exponential or polynomial function. Based on the parameterized curve, characteristic properties of chest non-linearity can be assessed. These properties can be compared to predetermined thresholds for acceptable compression magnitude and used as a basis to give feedback to the rescuer.

In one embodiment the invention comprises providing an indication of efficient chest compression based on the results of linearity analysis, where determination of non-linearity leads to indication of efficient chest compression and determination of linearity leads to indication of non-efficient chest compression.

The feedback system can for instance be integrated in a defibrillator or be a stand-alone system. Feedback can for instance be verbal or visual.

Values which can be derived from the non-linear function are:

a) Depth threshold for adequate compression magnitude

b) Force threshold for adequate compression magnitude

c) Depth or force threshold for feedback on incomplete release (leaning).

d) Rib break or collapse of the chest

e) Patient group (pediatric or adult)

f) Presence of a mattress underneath the patient.

a) A depth threshold for adequate compression magnitude can for instance be given by the depth at which the stiffness or incremental stiffness of the chest attains a certain value, or reaches a certain factor of the stiffness as measured at some predefined depth. This depth is likely to be much lower for small chests than for large chests. Thus, this measure can also be used to estimate the size of a patient's chest. In an embodiment of the invention, adequate compression depth is indicated by the system if the calculated stiffness k(x) at the obtained depth is more than twice as high as the stiffness measured for low deflections, for instance up to 10-15 mm depth.

The depth at which this occurs will here be termed the stiffening point of the chest. Additional criteria can also be applied to modify or override this threshold, for instance that minimum 38 mm depth and minimum 20 kg force shall be required to indicate adequate compression depth.

In another embodiment, adequate compression magnitude may be indicated when the non-linear part of the depth (see figure 2), exceeds a certain threshold, for instance 20 mm, and a 'press deeper' message may be issued when the non-linear depth is below the threshold.

b) A force threshold for adequate compression magnitude can for instance be related to the non-linear portion of the force, or in other words the force which causes non-linear displacement of the compression member. This can be expressed as the measured force minus the linear extrapolation of the force-displacement curve for low deflections. Thus, a "press harder" feedback message may be given if the non-linear portion of the force does not exceed a predefined value, for instance 10 kg. The linear portion of the force can be assessed by determining the chest stiffness for small compression depths, for instance the stiffness calculated at 10 mm compression depth.

As mentioned before, the clinical relevance of this criterion is that the linear part of the force can be assumed to stem from bending of the rib cage. Thus, it is only the remaining, non-linear part of the force which actually presses on the heart and thus generates blood pressure. If the non-linear portion of the force is lower than a certain limit, it can be expected that the chest compressions are not efficient in generating the necessary blood pressure and flow.

Figure 2 shows a schematic drawing of how the non-linear part of the force is defined.

Instead of force, the pressure on the chest may be estimated and be a basis for giving feedback. The pressure on the chest may be estimated by dividing the force by a typical chest area. This chest area may be modified to adapt the characteristics of each individual by assuming that the chest area is proportional to e.g.

the square of the chest size as estimated under point a) above.

c) A threshold for giving feedback on leaning can for instance be based on a portion of the force needed to reach a certain depth or force, or to reach a certain level of non-linearity. In an embodiment, leaning feedback or indication on leaning is given whenever the minimum force on the chest between two compressions is more than 10% of the force needed to reach a specific compression point. This point can for instance be the depth x where the calculated stiffness k(x) becomes more than twice as high as the stiffness measured at 15 mm depth.

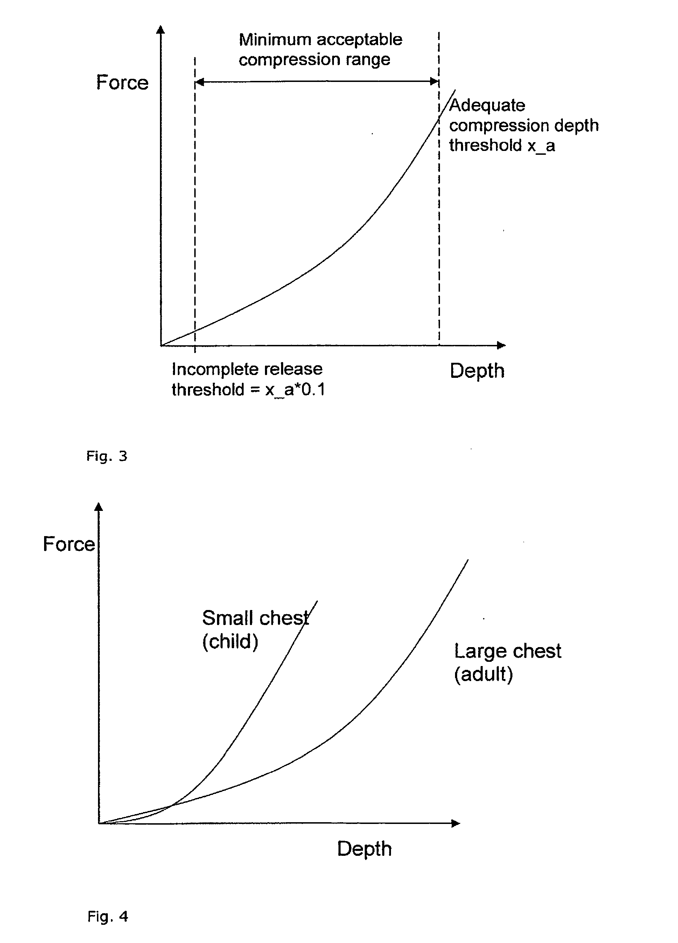

Figure 3 shows how, by choosing a leaning threshold as a portion of the depth or force required to reach an adequate compression magnitude, a minimum range of compression can be defined. Appropriate corrective feedback is given whenever the compression does not exceed the indicated range in either direction. The adequate depth threshold can for instance be defined as the stiffening point of the chest.

d) During CPR, ribs may break or the chest may literally collapse due to the mechanical impact of the compressions. This may result in a softening of the chest for low deflections. At the same time, a chest collapse will shift the neutral point of the chest downwards, and tend to increase the force for the same stroke length at high compression levels. In combination or separately these two effects may lead to a higher chest progressivity which may be detected by the system and used to give appropriate feedback or modify feedback thresholds.

e) A criterion for detecting the patient category with regards to age (adult or pediatric) can for instance be based on comparing the measured progressivity to typical properties for the various age groups. Using their definition of progressivity, Tomlinson et al (2006) found that the progressivity of adults was typically 1.4, with very few being over 2. Since children are smaller softer, they are expected to have a higher progressivity, In piglets with properties resembling children of 2-3 years, we have measured the typical progressivity to around 2. By measuring the progressivity of the patient, or in any other manner determining the non-linearity of the force/depth relationship e.g. as mentioned in items 2) and 3) above, it is possible to determine if the patient is a child or an adult. This information may be used to change settings between pediatric and adult settings.

Figure 4 shows typical non-linear force/depth relationships for adults and children. Children will typically have lower stiffness for low deflections, but the stiffness will increase much more rapidly with depth. By comparing measures of chest non-linearity, for instance the stiffening point, to typical ranges for children and adults, it can be determined if the patient is a child or an adult.

f) A criterion for distinguishing between subjects lying on surfaces with different stiffness and e.g. detecting the presence of a mattress underneath the patient can be based on the parameters characterizing the progressivity of the chest. Typically, the measured force/depth relationship of a body lying on a mattress will be linear (stiffness constant) up to much higher depths than when lying on a firm surface. The stiffness of the surface under the patient can thus be determined to a certain degree by the device according to the invention. Thus, if the measured progressivity of the chest, for instance the ratio of the force needed to reach 38 mm to the force needed to reach 19 mm depth is significantly lower than typical values for a body on a firm surface, the detection system will raise a flag that the person is probably lying on a mattress. Based on this information, the feedback system may give the user a warning that the patient is lying on a mattress and that feedback on depth may be uncertain.

If a mattress is detected, the system may alternatively switch to another mode of giving feedback, for instance from a depth criterion to a force based criterion where the Linear part of the curve is subtracted. In case of a mattress, this will be a relevant criterion because mattresses are often very linear in their characteristics.

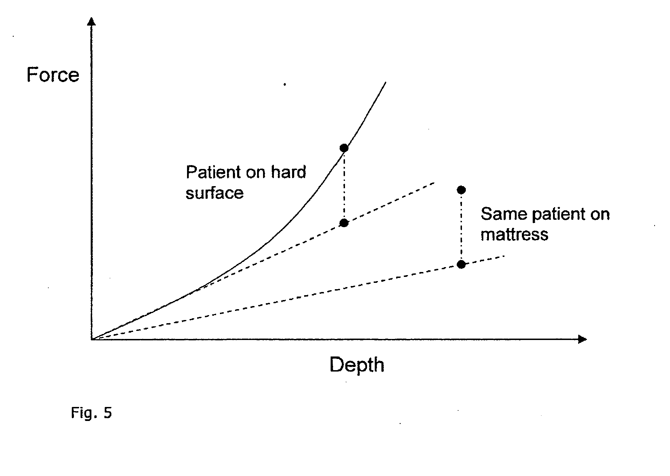

Figure 5 illustrates how, when a patient is placed on a mattress, a large part of the measured compression depth will be due to compression of the mattress and not deflection of the chest. Since the mattress typically has a linear force/depth profile, the non-linear part of the force or depth will however be approximately the same for the same level of chest deflection. By basing the feedback on non-linear parameters instead of depth, the effect of the mattress is thus cancelled out.

Additional information can be obtained by calculating the moving mass of the chest. For instance, if there is a mattress beneath the patient, the moving mass will in general be higher since the whole body is moving up and down in the bed. The estimated mass can then be used as an alternative, supplementary criterion to give feedback on the presence of a mattress Mass can be calculating by correlating the force and acceleration, specifically at the beginning of a compression where both the damping and elastic forces are low.

## Claims

1. Device arranged for monitoring chest compressions performed by means of a compression member, comprising a first sensor arranged for measuring force exerted by the compression member, a second sensor arranged for measuring displacement of the compression member, and a processor arranged for providing a chest stiffness function representing the relationship between force and displacement based on values derived from the measured force and the measured displacement, and for analyzing linearity of the chest stiffness function, **characterised in that** the device further comprises means for providing an indication of the chest compression efficacy based on the results of said linearity analysis, where the degree of non-linearity determines if a chest compression is indicated as efficient or non-efficient.

2. Device according to claim 1, **characterised in that** it comprises means for providing an indication of category based on the results of said linearity analysis, where the degree of non-linearity determines the category.

3. Device according to claim 1, **characterised in that** a non-efficient compression is indicated when the non-linear portion of the force does not exceed a predetermined value.

## Patentansprüche

1. Vorrichtung zum Überwachen von Thoraxkompressionen, durchgeführt mittels eines Kompressionsglieds, aufweisend einen ersten Sensor zum Messen einer durch das Kompressionsglied ausgeübten Kraft, einen zweiten Sensor zum Messen einer Verlagerung des Kompressionsglieds und einen Prozessor zum Bereitstellen einer Thoraxsteifigkeitsfunktion, die die Beziehung zwischen Kraft und Verlagerung abbildet, basierend auf von der gemessenen Kraft und der gemessenen Verlagerung abgeleiteten Werten, und zum Analysieren der Linearität der Thoraxsteifigkeitsfunktion, **dadurch gekennzeichnet, dass** die Vorrichtung des Weiteren Mittel zum Bereitstellen einer Indikation der Thoraxkompressionseffizienz umfasst, basierend auf den Ergebnissen der Linearitätsanalyse, wo der Grad der Nichtlinearität bestimmt, ob eine Thoraxkompression als effizient oder ineffizient angezeigt wird.

2. Vorrichtung gemäß Anspruch 1, **dadurch gekennzeichnet, dass** sie Mittel zum Bereitstellen einer Kategorieindikation umfasst, basierend auf den Ergebnissen der Linearitätsanalyse, wo der Grad der Nichtlinearität die Kategorie bestimmt.

3. Vorrichtung gemäß Anspruch 1, **dadurch gekennzeichnet, dass** eine ineffiziente Kompression indiziert wird, wenn der nichtlineare Anteil der Kraft einen festgelegten Wert nicht über-

schreitet.

## Revendications

1. Dispositif agencé pour surveiller des compressions thoraciques effectuées au moyen d'un élément de compression, comprenant un premier capteur agencé pour mesurer la force exercée par l'élément de compression, un deuxième capteur agencé pour mesurer le déplacement de l'élément de compression, et un processeur agencé pour fournir une fonction de rigidité thoracique représentant la relation entre la force et le déplacement sur la base de valeurs déduites de la force mesurée et du déplacement mesuré, et pour analyser la linéarité de la fonction de rigidité thoracique, **caractérisé en ce que** le dispositif comprend en outre des moyens pour fournir une indication de l'efficacité de la compression thoracique sur la base des résultats de ladite analyse de linéarité, où le degré de non linéarité détermine si une compression thoracique est indiquée comme étant efficace ou non efficace.

2. Dispositif selon la revendication 1, **caractérisé en ce qu'**il comprend des moyens pour fournir une indication de catégorie sur la base des résultats de ladite analyse de linéarité, où le degré de non linéarité détermine la catégorie.

3. Dispositif selon la revendication 1, **caractérisé en ce qu'**une compression non efficace est indiquée lorsque la partie non linéaire de la force ne dépasse pas une valeur prédéterminée.

Force

Actually measured
force/depth curve

Parametrized force/depth
curve

Nonlinear part of force/depth curve

Linear part of force/depth curve

Depth

Fig. 1

force

Non-linear depth

Total force

Non-linear force

Linear force

10 mm    depth

Fig. 2

Fig. 3

Fig. 4

Fig. 5

**REFERENCES CITED IN THE DESCRIPTION**

**Patent documents cited in the description**

- EP 1491176 A1

- WO 0215836 A